# EUROPEAN PATENT APPLICATION

(11) **EP 1 269 936 A2**
(43) Date of publication of application: **02.01.2003**
(21) Application number: 02013879.8
(22) Date of filing: 24.06.2002
(51) Int. Cl.: A61F 2/08

(54) **A ligament fixation device**

(30) Priority: 29.06.2001 GB 0115940
(71) Applicant: Atlantech Medical Devices Limited, Harrogate HG3 1DH (GB)
(72) Inventor: Seyr, Volkmar, 6060 Ampass (AT); Goreis, Helmut, 6020 Innsbruck (AT)
(74) Representative: Walsh, David Patrick

(57) **Abstract**

The invention relates to a ligament fixation device for the fixation of an anterior cruciate ligament in the knee joint which device can be inserted into appropriately drilled holes in the femur and/or tibia to locate the replacement therein. The device comprises an outwardly expandable compression member and a pair of anchoring members. The compression member has a first surface and a second surface, spaced from the first surface and joined to the first surface by a side wall. The side wall of the compression member extends from a narrower first surface to a wider second surface. The wall of the compression member includes a plurality of spaced slits. A first anchoring member is associated with the first surface of the compression member and a second anchoring member is associated with the second surface of the anchoring member so that the three members are located sequentially along a common axis. Means to urge the anchoring members into and maintain them at closer axial proximity is provided to thereby compress the compression member and cause outward expansion thereof.

## Description

The present invention relates to a ligament fixation device and, in particular, but not exclusively, to a ligament fixation device for the fixation of an anterior cruciate ligament in the knee joint which device can be inserted into appropriately drilled holes in the femur and/or tibia to locate the replacement therein.

Due to increasing involvement of people with active sport, injuries are becoming increasingly common where tissues such as ligaments or tendons tear or detach from bone. Surgical techniques have been developed to reconstruct such torn soft tissues and to re-attach them to the relevant bone. One of the most common types of such injuries is tearing of the Anterior Cruciate Ligament (ACL). The Anterior Cruciate Ligament connects the femur to the tibia at the centre of the knee joint. Reconstruction of such tissues generally involves replacement with a graft such as autologous or artificial tendon. An autologous tendon graft may be taken from the patients patellar tendon or, alternatively, the semitendinosus may be utilised. A typical fixation technique involves the use of a circular button fixation device which is located on the outside of the femur above the knee. As this is some distance from the site where the graft will be utilised in the knee joint, sutures are used to attach the graft to the femur button. The main disadvantage of this technique is that incisions need to be made through the skin and quadriceps muscle resulting in trauma to the leg and a cosmetically undesirable procedure.

Use of the endobutton also involves laborious measurement of lengths and drilling with two drill diameters.

US 5645588 describes an improved technique whereby the ligament anchor may be threaded through a femoral tunnel formed through the femur from the centre of the knee.

PCT/US97/22061 provides an interference fit insertion element with a proximal aperture through which a graft may be threaded or attached. The technique involves securement of the interference end of the device into the central cancellous area of the bone.

US 5037422 also relates to the anchoring of sutures for securement of a suture to a bore hole in a bone. The device is shown secured to the cancellous bone. The device is for use in soft tissue fixation to the outside of the bone and not fixation within the bone hole itself.

A device for graft fixation using sutures is described in EP 0 619 982 where the anchor includes a body and a plurality of barbs located in axially aligned, circumferentially spaced relation to each other about the body. The barbs have a normal configuration wherein they extend rearwardly and radially outwardly from the anchor body to outer ends which are normally located outside a longitudinal projection of the largest geometric cross-section of the body transverse to its longitudinal axis. The device relies upon interference between the barbs and the soft cancellous bone area. This interference fit is not only difficult to insert for reasons of space but also involves some risk of damage to the transplant.

Interference fit bone anchors are, therefore, increasingly common for ligament fixation. A problem for the surgeon fitting the device is how to insert the device without the interfering projections impeding the initial location in the closely fitting bone hole. EP 1066805 solves this problem by using a cruciform device which expands at the distal end when the replacement ligament is tensioned ie. after the cruciform anchor has been located in position.

It is an object of the present invention to provide an alternative bone anchor with interfering projections.

Recently, a "rigid fix" system has been developed. However, the "rigid fix" system is relatively costly to install and requires further procedures. Furthermore, the length of time for installation is inconvenient.

It is a further object of the present invention to provide a device which is easier to implement than the rigid fix.

According to a first aspect of the present invention there is provided a ligament fixation device comprising an outwardly expandable compression member, a pair of anchoring members, the said compression member having a first surface and a second surface, spaced from the first surface and joined to the first surface by a side wall, the side wall of the compression member extending from a narrower first surface to a wider second surface, the said wall of the compression member between the said first and second surfaces including a plurality of spaced slits extending partially along the said wall from the second surface and in the direction of the first surface, the compression member being hollow so that compression of the compression member causes outward expansion of the said wall of the compression member, the said anchoring members being located on either side of the compression member, wherein a first anchoring member is associated with the first surface of the compression member and a second anchoring member is associated with the second surface of the anchoring member so that the three members are located sequentially along a common axis and means to urge the anchoring members into and maintain them at closer axial proximity to thereby compress the compression member and cause outward expansion thereof.

Preferably, the said outward expansion is beyond the outer lateral limit of the said anchoring members.

Preferably, the means to urge the anchoring members into closer axial proximity is an adapted central shaft which passes centrally through the two anchoring members and the compression member for relative multiple position fixation of the said anchoring members.

Preferably, the multiple position fixation is provided by threaded engagement between at least one of the anchoring members and the said shaft.

Both anchoring members may be threadedly engaged with the central shaft or one may be fixed eg. as a head of the shaft. In either case, the compression member is preferably, axially slideable on the shaft via a centrally disposed aperture.

When both anchoring members have central threaded holes, the shaft may include a threaded portion and a handle portion joined to the threaded portion by a frangible section. The frangible section is, preferably, designed to break at a predetermined tension of the compression member. Preferably, the frangible section is located close to the limit of insertion of the threaded shaft in the bone hole so that breakage of the frangible section leaves the threaded portion in position in the bore hole whilst the handle can be removed.

Preferably, the compression member and anchoring member are all circular in end section. Preferably, the shaft is also circular in end section as are the centrally disposed apertures in each of the compression member and anchoring members. In such embodiments, the slits in the wall of the compression member are in a radial direction and begin at the second surface. One of the anchoring members may include a drilled hole or an adaptation to receive a suture or transplant at its free end ie. that and not associated with the compression member, to fix the transplant in position.

Preferably, the fixation device is in the form of a cylinder in three sections. In use, the transplant may be located behind the cylinder or may be suspended in front of it. Preferably, it is suspended in front of it.

Preferably, rotation of the threaded shaft urges the threaded anchoring members into closer axial proximity thereby causing compression and expansion of the compression member beyond the outer limit of the anchoring members and towards the wall(s) of the bone hole to provide secure fixation.

A ligament fixation device comprising a threaded shaft, an expandable washer located on the shaft between a first nut and second member which may be a further nut or a bolt head for the threaded shaft so that rotation of the shaft urges the nut and second member together causing compression and expansion of the washer beyond the outer radial limit of the nut and second member.

Advantageously, after location of the fixation device in the bone hole and alignment of the transplant, expansion of the compression member causes its outer edge to be urged into engagement with the wall of the bone hole thereby fixing the transplant into position.

Grooves may be provided on the compression member to prevent damage to the transplant.

Preferably, a series of compression members may be provided. Preferably, each compression member is interposed between respective anchoring members so that a repeat sequence of anchoring member then compression member is formed. Alternatively, a series of non-expandable slideably axis engageable spacers may be located between successive compression members with anchoring members being only located at either axial end.

According to a second aspect of the present invention there is provided a method of ligament fixation comprising the steps of:
forming a bone hole in a joint suitable for ligament fixation;
locating a ligament fixation device according to the first aspect of the invention in the said bone hole together with a suitable transplant;
activating means to compress the compression member and therefore cause outward expansion thereof sufficient to securely fix the transplant and the said device in the bone hole.

The transplant may be looped over the head of the fixation device or it may be located on one side thereof so that, in either case, expansion of the compression member urges the transplant against the wall of the bone hole.

The transplant may also include a bone-tendon-bone transplant and, in such cases, the bone section at one end of the transplant may be located behind the head of the fixation device to thereby prevent its longitudinal movement in the bone hole after the fixation device is secured.

Preferably, the compression means axially compresses the compression member.

Examples of the invention will now be described with reference to the accompanying drawings in which:-
Figure 1 shows a part sectional view of the components of a device in accordance with the invention;
Figure 2 shows a plan view of a compression member in accordance with the present invention;
Figure 3(a) -(c) show methods of transplant fixation in accordance with the present invention.

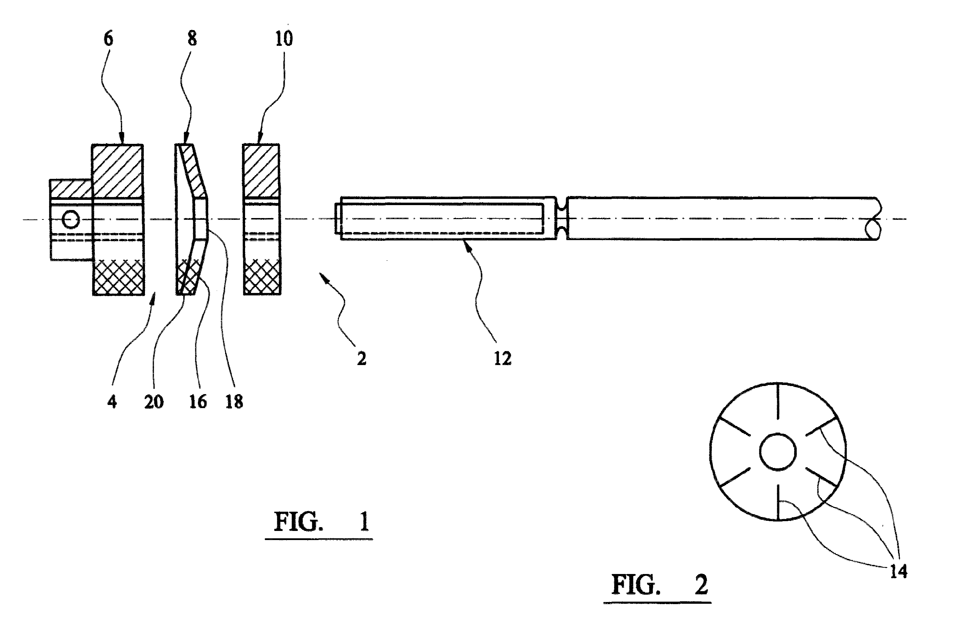

Referring to figure 1, a ligament fixation device 2 is shown which consists of a larger cylinder 4 in three parallel sections 6, 8 and 10. Each of the sections comprises a ring and each of the rings have the same diameter and include a centrally disposed aperture for shaft 12 to extend therethrough. The apertures in outer rings 6 and 10 are threaded for threaded engagement with the shaft 12 and the aperture of central ring 8 is unthreaded for slidable axial engagement with the shaft 12.

The outer lateral surface of each ring has been milled for ease of gripping by the surgeon and to increase fixation in the bone hole.

The central ring 8 is in the form of a hollow frustoconical member having a sloping side wall 16 extending from the central aperture 18 to the outer lateral edge 20. The wall 16 has a series of circumferentially spaced radially extending slits 14, which extend from the outer lateral edge 20 towards the central aperture 18 of the ring.

In use, the transplant is preferably located over the fixation device and the device is located in the bone hole. The shaft is then, preferably, rotated to bring the outer rings 6 and 10 into closer proximity thus causing expansion of the compression ring 8 in the bone hole to secure the anchor in position and, preferably, urge the transplant into contact with the walls of the bone hole.

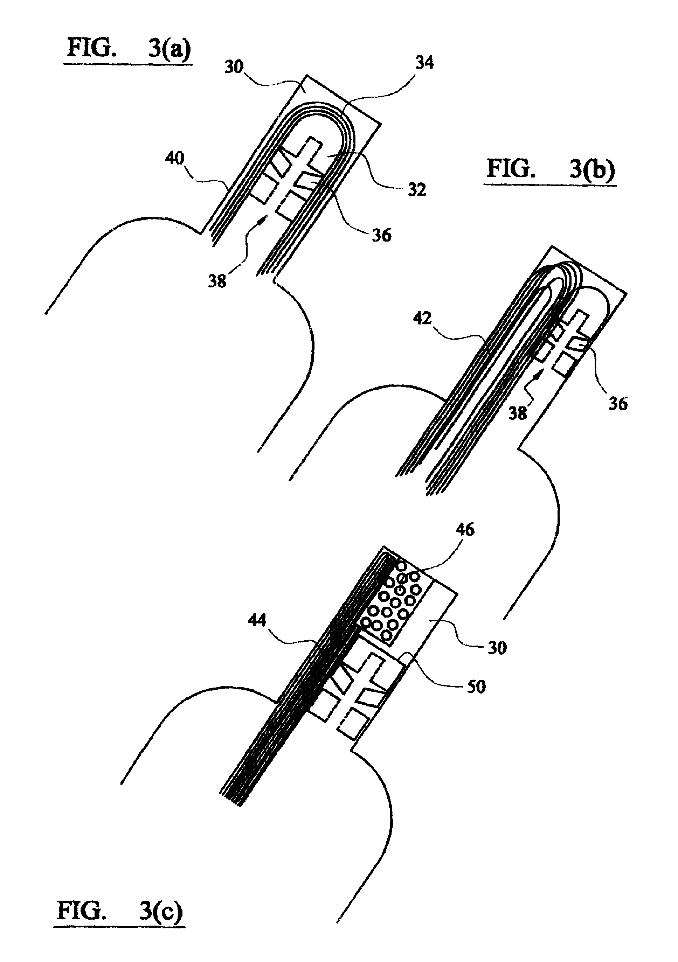

Referring to figure 3a-c, three different fixation methods are shown. In this case, the bone hole may be a femoral or tibial tunnel. Referring to figure 3a, the tunnel 30 has a fixation device similar to that previously described located therein. The head 32 of the device is bullet-shaped to allow the transplant 34 to be located thereover without damage to the transplant. In some embodiments, suitable grooves may be formed in the head 32 to locate the transplant in position. It can readily be seen that expansion of the compression member would urge the transplant into contact with the walls 40 of the bone hole 30 to thereby assist the grafting process and secure the fixation device 38 in the bone hole. Referring to figure 3b, the transplant 42 is located on one side of the fixation device 38. In this case, expansion of the compression ring causes expansion and urges the transplant into the wall of the bone hole whilst the other side of the compression member is urged into the wall of the bone hole to thereby secure transplant and the fixation device.

Referring to figure 3c, the transplant 44 is of the bone-tendon-bone type and the bone section 46 is shown at the blind end of the tunnel 30 with the transplant extending down on one side of the fixation device. The fixation device has a flat top 50 to match the flat mating surface of the bone section 46. Again, expansion of the compression member fixes the transplant and the fixation device thus securing the graft in position.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A ligament fixation device comprising an outwardly expandable compression member, a pair of anchoring members, the said compression member having a first surface and a second surface, spaced from the first surface and joined to the first surface by a side wall, the side wall of the compression member extending from a narrower first surface to a wider second surface, the said wall of the compression member between the said first and second surfaces including a plurality of spaced slits extending partially along the said wall from the second surface and in the direction of the first surface, the compression member being hollow so that compression of the compression member causes outward expansion of the said wall of the compression member, the said anchoring members being located on either side of the compression member, wherein a first anchoring member is associated with the first surface of the compression member and a second anchoring member is associated with the second surface of the anchoring member so that the three members are located sequentially along a common axis and means to urge the anchoring members into and maintain them at closer axial proximity to thereby compress the compression member and cause outward expansion thereof.

2. A ligament fixation device according to claim 1, wherein the said outward expansion is beyond the outer lateral limit of the said anchoring members.

3. A ligament fixation device according to claim 1 or 2, wherein the means to urge the anchoring members into closer axial proximity is an adapted central shaft which passes centrally through the two anchoring members and the compression member for relative multiple position fixation of the said anchoring members.

4. A ligament fixation device according to any preceding claim, wherein the multiple position fixation is provided by threaded engagement between at least one of the anchoring members and the said shaft.

5. A ligament fixation device according to any preceding claim, wherein the compression member is axially slideable on the shaft via a centrally disposed aperture.

6. A ligament fixation device according to any preceding claim, wherein when both anchoring members have central threaded holes, the shaft includes a threaded portion and a handle portion joined to the threaded portion by a frangible section.

7. A ligament fixation device according to claim 6, wherein the frangible section is designed to break at a predetermined tension of the compression member.

8. A ligament fixation device according to any preceding claim, wherein the compression member and anchoring member are all circular in end section.

9. A ligament fixation device according to claim 8, wherein the slits in the wall of the compression member are in a radial direction and begin at the second surface.

10. A ligament fixation device according to any preceding claim, wherein the fixation device is in the form of a cylinder in three sections.

11. A ligament fixation device according to any preceding claim, wherein rotation of the threaded shaft urges the threaded anchoring members into closer axial proximity thereby causing compression and expansion of the compression member beyond the outer limit of the anchoring members and towards the wall(s) of the bone hole to provide secure fixation.

12. A ligament fixation device comprising a threaded shaft, an expandable washer located on the shaft between a first nut and second member which may be a further nut or a bolt head for the threaded shaft so that rotation of the shaft urges the nut and second member together causing compression and expansion of the washer beyond the outer radial limit of the nut and second member.

13. A ligament fixation device according to any preceding claim, wherein guide grooves may be provided on the compression member to prevent damage to the transplant.

14. A ligament fixation device according to any preceding claim, wherein a series of compression members is provided.

15. A ligament fixation device according to claim 14, wherein each compression member is interposed between respective anchoring members so that a repeat sequence of anchoring member then compression member is formed.

16. A ligament fixation device according to claim 14, wherein one or more non-expandable slideably axis engageable spacers are each respectively located between successive compression members with anchoring members being only located at either axial end of the device.
